# EUROPEAN PATENT SPECIFICATION

(11) **EP 3 071 514 B1**
(45) Date of publication and mention of the grant of the patent: **13.03.2019**
(21) Application number: 14802650.3
(22) Date of filing: 24.11.2014
(51) Int. Cl.: B67D 7/34

(54) **METHODS AND SYSTEM FOR DETERMINING FUEL QUALITY IN A VEHICLE**
VERFAHREN UND SYSTEM ZUR BESTIMMUNG DER KRAFTSTOFFQUALITÄT IN EINEM FAHRZEUG
PROCÉDÉS ET SYSTÈME DE DÉTERMINATION DE QUALITÉ DE CARBURANT DANS UN VÉHICULE

(30) Priority: 22.11.2013 GB 201320643
(43) Date of publication of application: 28.09.2016
(73) Proprietor: Jaguar Land Rover Limited, Coventry, Warwickshire CV3 4LF (GB)
(72) Inventor: DHANDE, Kunal, Coventry Warwickshire CV3 4LF (GB); PISHNESHIN, Adel, Coventry Warwickshire CV3 4LF (GB)
(74) Representative: Hopley, Joanne Selina
(86) International application number: PCT/EP2014/075433
(87) International publication number: WO 2015/075244

(56) References cited:
- EP-A1- 2 037 112
- EP-A2- 1 134 574
- WO-A1-94/08226
- US-A1- 2004 069 273
- US-A1- 2012 078 487

## Description

### TECHNICAL FIELD

The present invention relates to a method and system for determining fuel quality in a vehicle during or following a refuelling stop. Aspects of the invention relate to a method, a system, a control module, an engine control unit, and a vehicle.

### BACKGROUND

Within the European Union and United States of America there have been significant moves in recent years to establish and maintain high standards for automotive fuels such as gasoline/petroleum (or "petrol") and diesel. Increasingly stringent criteria which liquid fuels must meet are dictated in part by a drive to reduce the environmental impact of motor vehicles and improve sustainability of remaining hydrocarbon resources. For example, since the year 2000, the marketing of petrol comprising additives such as tetraethyl lead has been banned within the EU for most vehicles. EU-wide Directives such as 98/70/EC and 2005/55/EC specify stringent environmental specifications with associated timelines for implementation to be applied to fuels for road vehicles and non-road mobile machinery, agricultural vehicles, and recreational craft when not at sea. Until 2013, for instance, suppliers must place on the market petrol with a maximum oxygen content of 2.7 % and a maximum ethanol content of 5 %. These rigid specifications allow for some variations between winter and summer fuels, but set very clear parameters for the maximum levels of environmental pollutants such as sulphur, which must not have exceeded 10mg/kg in diesel fuels from 1 January 2011. Indeed, the regulatory burdens upon suppliers of petrol within the EU are such that they are currently obliged to meet a requirement to gradually reduce life cycle greenhouse gas emissions by 10 % by 31 December 2020 at the latest. These Directives provide intermediary objectives for the course of this time period. In order to ensure compliance with EU and equivalent US regulations most modern fuels contain an increasing proportion of biofuels obtained from diverse sources such as wood, algae and crops. The biofuel content of a fuel may vary from supplier to supplier and region to region. For example, even within the US the biofuel levels within diesel fuel can vary between states from around 5%wt to as much as 20%wt of the fuel. It has been noted that biofuel content in diesel is generally higher in the Mid-Western States of the US.

It is evident that the drives to reduce the environmental impact of fossil fuel use, particularly in relation to automotive fuels, have led to improved and more sophisticated design of fuels within the US and EU. Modern automotive fuels are complex formulations of base fuel and additives, including dispersants, detergents, lubricants and other combustion control agents. The intention for fuel formulators is not just to meet regulatory requirements but also to create fuel formulations that optimise vehicle performance, reduce the cost of motoring, reduce engine wear and prolong the effective working life of the vehicle. Within such an environment it is understandable that automotive, and especially engine, manufacturers have also focussed their attentions on producing vehicles that are optimised to perform at their best with these new advanced fuel formulations. In addition, for engines that run on diesel fuels there are often statutory requirements for on-board diagnostic systems that ensure that during operation the engine functions within the regulatory emissions parameters. However, in a truly global marketplace automotive manufacturers have to meet significant demand in markets that are outside of the EU and US. In many territories, such as in Asia, Africa or South America the fuels available to the consumer might regularly fall below the standards required in more heavily regulated markets such as the EU. In some locations, adulteration of fuel supplies with cheaper hydrocarbon sources such as duty free heating oils like kerosene is commonplace, particularly where policing of fuel contamination is poor. This presents a significant challenge for automotive makers who aim to manufacture a consistent product that can meet the stringent emissions and efficiency requirements of the EU or US, but can also cope with the challenges of operating in less regulated markets.

Document EP2037112 describes a prior art system for determining the nature of a fuel that is being injected into an engine. Document US2004/069273 describes a prior art system including an acoustic wave sensor for measuring viscosity and density of a gasoline fuel so as to predict engine combustion quality during an engine start.

Therefore, against this background it is an aim of the present invention to provide an improved system and apparatus for monitoring any variation in fuel quality within a vehicle and to provide an alert to the operator when fuel quality is poor following refuelling of the vehicle. These and other uses, features and advantages of the invention should be apparent to those skilled in the art from the teachings provided herein.

### SUMMARY OF THE INVENTION

According to an aspect of the invention there is provided a method for determining whether fuel quality falls below a predetermined quality threshold during or after a vehicle refuelling event, comprising the steps of:
a) determining that a novel fuel has been introduced into a vehicle;
b) undertaking at least one measurement of the novel fuel in order to determine at least one first parameter of the novel fuel, wherein the first parameter is associated with a physical and/or a chemical property of the fuel;
c) calculating the value of at least one second parameter based upon the first parameter determined in step b, the value of the at least one second parameter being determined by comparison to fuel survey information;
d) comparing the first and second parameters of the novel fuel with corresponding threshold reference parameters in order to determine whether fuel quality falls below a predetermined quality threshold; and
e) providing an alert to the operator of the vehicle to indicate whether the fuel quality of the novel fuel falls below the predetermined quality threshold,
wherein the at least one first parameter includes density and the at least one second parameter includes viscosity or aromatics, and/or
wherein the at least one first parameter includes cetane number and the at least one second parameter includes aromatics, and/or
wherein the at least one first parameter includes biodiesel content and the at least one second parameter includes viscosity.

The at least one first parameter of the novel fuel may be associated with a property of a particular fuel type. The at least one first parameter of the novel fuel may be associated with a property of an expected fuel type. The at least one first parameter of the novel fuel may be used to distinguish a level of quality within a particular fuel type. The predetermined quality threshold may be a threshold for a particular fuel type. Fuel types may include gasoline-based fuel and diesel-based fuel. The predetermined quality threshold may be determined by a national or regional standard for a particular fuel type. The standard may be specified within a national or regional directive or legislation. Where a national or regional standard specifies an allowable range for a parameter of the novel fuel, the predetermined quality threshold may lie between the limits of the range.

A method may be provided for determining whether fuel quality falls below a predetermined quality threshold for a gasoline fuel during or after a vehicle refuelling event, comprising the steps of:
(A) determining that a novel fuel has been introduced into a vehicle;
(B) undertaking at least one measurement of the novel fuel in order to determine at least one first parameter of the novel fuel, wherein the first parameter is associated with a property of the fuel;
(C) calculating the value of at least one second parameter based upon the first parameter determined in step B, the value of the at least one second parameter being determined by comparison to fuel survey information;
(D) comparing the first and second parameters of the novel fuel with corresponding threshold reference parameters in order to determine whether fuel quality falls below a predetermined quality threshold; and
(E) providing an alert to the operator of the vehicle to indicate whether the fuel quality of the novel fuel falls below the predetermined quality threshold,
wherein the at least one first parameter includes density and the at least one second parameter includes viscosity or aromatics, and/or wherein the at least one first parameter includes cetane number and the at least one second parameter includes aromatics, and/or wherein the at least one first parameter includes biodiesel content and the at least one second parameter includes viscosity.

A method may be provided for determining whether fuel quality falls below a predetermined quality threshold for a diesel fuel during or after a vehicle refuelling event, comprising the steps of:
(I) determining that a novel fuel has been introduced into a vehicle;
(II) undertaking at least one measurement of the novel fuel in order to determine at least one first parameter of the novel fuel, wherein the first parameter is associated with a property of the fuel;
(III)calculating the value of at least one second parameter based upon the first parameter determined in step II, the value of the at least one second parameter being determined by comparison to fuel survey information;
(IV)comparing the first and second parameters of the novel fuel with corresponding threshold reference parameters in order to determine whether fuel quality falls below a predetermined quality threshold; and
(V) providing an alert to the operator of the vehicle to indicate whether the fuel quality of the novel fuel falls below the predetermined quality threshold,
wherein the at least one first parameter includes density and the at least one second parameter includes viscosity or aromatics, and/or
wherein the at least one first parameter includes cetane number and the at least one second parameter includes aromatics, and/or
wherein the at least one first parameter includes biodiesel content and the at least one second parameter includes viscosity.

The diesel fuel may be light duty diesel fuel.

According to one embodiment of the invention, the method is initiated by opening of a fuel flap, removal of a fuel cap or by commencing a refuelling event.

In an embodiment of the invention the property of the fuel is selected from a physical and/or a chemical property of the fuel.

In a specific embodiment of the invention the fuel comprises a gasoline. In an alternative embodiment of the invention the fuel comprises a diesel fuel. Optionally the fuels may comprise a biofuel component, which may include ethanol.

In embodiments of the invention where the fuel comprises a gasoline, according to the invention the one or more parameters may be selected from one or more of the group consisting of: temperature, RON index; Oxygen content; volatility (e.g. Reid vapour pressure); ethanol content and density.

In embodiments of the invention where the fuel comprises a diesel fuel, according to the invention the one or more parameters may be selected from one or more of the group consisting of: temperature; heating value; biodiesel content; Cetane number/index; density aromatics content; sulphur content; water content; fatty acid methyl ester (FAME) content; and wax content.

According to a specific embodiment of the invention at least two parameters are determined in step (b), suitably at least three parameters; optionally at least five parameters are determined; in some embodiments five or more parameters are determined.

A determination of whether the novel fuel falls below the predetermined quality threshold may comprise comparing each of said at least two first parameters with a respective plurality of threshold reference parameters.

The plurality of threshold reference parameters may be indicative of fuel which is in compliance with a regulatory standard.

The determination that a novel fuel has been introduced into the vehicle may be made in response to a positive determination that the amount of fuel stored in the vehicle has increased since a previous deactivation of the vehicle engine.

The determination that a novel fuel has been introduced into the vehicle may be made in dependence on a residual amount of fuel stored in the vehicle prior to a detected increase in the amount of fuel stored in the vehicle.

A positive determination that a novel fuel has been introduced into the vehicle may be made only when said residual amount of fuel is less than a threshold amount.

The threshold amount may in the range of between about 20% to 80% of the total capacity of a fuel storage tank of the vehicle, and suitably in a range between about 30% and 70%, and more suitable in the range between about 40% and 60%.

In a specific embodiment of the invention the alert comprises one or more of the group selected from the group consisting of: a warning light; a warning icon displayed on a high level display front (HLDF); playback of a recorded audio message; and a more detailed diagnostic report message displayed on an HLDF.

In specific embodiments of the invention the comparison step (d) occurs within a microprocessor device that may comprise a memory.

Another aspect of the invention provides a system for use in a vehicle which system determines whether fuel quality falls below a predetermined quality threshold during or after a vehicle refuelling event, the system comprising:
(1) at least one sensor, the sensor being adapted so as to undertake at least one measurement of a novel fuel that has been introduced into the fuel system of the vehicle in order to determine at least one first parameter of the novel fuel, wherein the first parameter is associated with a physical and/or a chemical property of the fuel;
(2) at least one processor that is in communication with the sensor, and which calculates the value of at least one second parameter based upon the first parameter, the value of the at least one second parameter being determined by comparison to fuel survey information, and compares the at least one first parameter and the at least one second parameter of the novel fuel with corresponding threshold reference parameters in order to determine whether fuel quality falls below a predetermined quality threshold; and
(3) a display that is in communication with the at least one processor for providing an alert to an operator of the vehicle to indicate whether the fuel quality of the novel fuel falls below the predetermined quality threshold,
wherein the at least one first parameter includes density and the at least one second parameter includes viscosity or aromatics, and/or
wherein the at least one first parameter includes cetane number and the at least one second parameter includes aromatics, and/or
wherein the at least one first parameter includes biodiesel content and the at least one second parameter includes viscosity.

The invention also extends to an engine control unit (ECU) comprising a control module that operates a method according to the above-described aspects of the invention, a vehicle comprising a control module according to the above-described aspects, and a vehicle comprising a system according to the above-described aspect. The invention further extends to a vehicle comprising said engine control unit.

Within the scope of this application it is expressly envisaged that the various aspects, embodiments, examples and alternatives set out in the preceding paragraphs, in the claims and/or in the following description and drawings, and in particular the individual features thereof, may be taken independently or in any combination. Features described in connection with one embodiment are applicable to all embodiments, unless such features are incompatible.

### BRIEF DESCRIPTION OF THE DRAWINGS

Embodiments of the present invention will now be described, by way of example only, with reference to the accompanying drawings, in which like components are assigned like numerals, and in which:-
Figure 1 is a schematic diagram of a vehicle comprising a system of the invention (a) shows a first embodiment of the invention, (b) shows a second embodiment of the invention;
Figure 2 a flow diagram illustrating a method according to one embodiment of the invention;
Figure 3 is a schematic illustration of a sensor according to one embodiment of the invention;
Figure 4 is a graph showing a correlation between density and viscosity for diesel fuel survey composition data;
Figure 5 is a graph showing a correlation between 95% distillation temperature and density for diesel fuel survey composition data;
Figure 6 is a graph showing a correlation between cetane number and density for diesel fuel survey composition data; and
Figure 7 is a graph showing a correlation between aromatics content and cetane number for diesel fuel survey composition data.

### DETAILED DESCRIPTION

Prior to setting forth the invention, a number of definitions are provided that will assist in the understanding of the invention.

The term "vehicle" as used herein includes any conveyance, or model of a conveyance, where the conveyance was originally designed for the purpose of moving one or more tangible objects, such as people, animals, cargo, under motive power. Typical vehicles may include but are in no way limited to cars, trucks, lorries, motorcycles, buses, automobiles, trains, railed conveyances, boats, ships, marine conveyances, aircraft and the like.

The term "fuel" is used herein to denote hydrocarbon based fluids - typically liquids - that are used as the basis for an internal combustion reaction within an automotive engine. Suitably the internal combustion engine is of the spark ignition or compression ignition type. For spark ignition engines the fuel will suitably comprise gasoline or refined petroleum. Gasoline may be supplemented with a minor or even major component of ethanol in some cases derived from biological sources. For compression ignition engines the fuel will comprise diesel fuel. Diesel fuel may be supplemented with a minor or major component of biofuel such as a vegetable-derived or oilseed based biodiesel.

As used herein the term "fuel quality" refers to a number of parameters that contribute to the efficiency and energy rating of hydrocarbon fuels used in spark or compression ignition engines. Higher quality fuels will enable engines to operate with greater energy efficiency, producing fewer harmful emissions for longer periods and with reduced wear. For gasoline-based fuels factors such as the so-called octane rating - which measures the fuel's resistance to autoignition via comparison with a standard mixture of 2,2,4-trimethylpentane to give a research octane number (RON) number - as well as other parameters such as density and content of oxygenates and aromatics, are considered important in determining fuel quality. Compression ignition fuels, such as diesel fuels, are assessed for quality via parameters such as heating value, cetane number (see below), density as well as content of aromatics, wax (cloud point), and biodiesel. Biodiesel content is important to determine as many biodiesels comprise fatty-acid alkyl ester (FAAE) compounds which can cause degradation of rubber and polymer based engine components that come into contact with the fuel, especially seals and gaskets. Adverse fuel quality may be defined as fuel exhibiting properties or parameters that fall below certain benchmark performance levels - which may include EU or US standards - or fuel quality that may cause either long term loss of efficiency or damage to the engine, or even acute engine failure. It should be noted however, that the determination of fuel quality is distinct and different from the assessment of whether the correct fuel has been introduced into the vehicle. For example, the effects of chronic use of low quality diesel or gasoline fuel on an engine are different and distinct from the acute effect of mistakenly fuelling a diesel equipped vehicle with gasoline. The present invention is directed primarily at the former case in point rather than the latter.

The term "cetane number" or "CN" as used herein denotes the measurement of a compression ignition (e.g. diesel) fuel's ignition delay, which corresponds to the time interval between the start of an injection cycle and the first identifiable pressure increase during combustion of the fuel. In a given diesel engine, higher cetane number fuels demonstrate shorter ignition delay periods when compared to fuels having a lower cetane number. The current standard for diesel sold in the EU, Iceland, Norway and Switzerland is set in EN 590, with a minimum cetane index of 46 and a minimum cetane number of 51. Premium diesel fuel can have a cetane number as high as 60. Understandably, many factors contribute to the cetane number of a given diesel fuel some of which may derive from the additive package comprised within the fuel itself. However, the cetane number and, thus, fuel quality will be reduced when low energy value hydrocarbons, some biofuels and adulterants are included in the fuel supply.

The term "refuelling event" as used herein refers to the occasion where a new or novel fuel composition is introduced into the fuel line of a vehicle. As mentioned previously fuel compositions typically comprise a diverse mixture of components. As such, a refuelling event is defined as the time point at which the fuel comprised within the fuel line exhibits a detectable change in composition. Usually this change is caused by the need to take on-board additional fuel following depletion of existing fuel stocks through normal operation of the engine. Hence, the refuelling event may involve introduction of up to 100% of an entirely new fuel load, such as in instances where the fuel is drained from the engine and entirely replaced. However, more often the refuelling event will involve introduction of less than 100% of the fuel load, as the operator 'fills up' the vehicle. In these instances the newly introduced fuel will mix fully or partially with the residual fuel load in order to form a mixed fuel load that typically will exhibit at least some changed chemical or physical properties (dependent upon the source of the fuel). The resultant hybrid fuel mixture can be considered as representing a new fuel composition.

The term "measurement" as used herein refers to the assessment or determination of at least one parameter associated with a given fuel. Typically the at least one parameter will be associated with or contributory to an assessment of fuel quality. Suitably, at least two parameters may be determined as part of a measurement. The at least two parameters may be linked in some way so that other parameters may be determined or calculated as a result of knowledge of the at least two parameters. Even more suitably, the measurement may include determination of a plurality of parameters associated with a given fuel under test. Optionally, the number of parameters determined may be more than three, more than five or more than six. Alternatively, the number of parameters comprised within the measurement may be less than ten, or at most five. The parameters that are assessed for any given fuel may include one or more of the following non-limiting examples:
- Heating value
- Density
- Temperature
- Viscosity
- Lubricity
- Volatility (e.g. Reid vapour pressure)
- RON index
- Cetane number/index
- Ethanol content
- Sulphur content
- Oxygen content
- Water content
- Aromatics content
- Biodiesel content
- FAAE content
- Wax content (e.g. cloud point)
- Cold flow

The term "sensor" as used herein refers to a device - or a series of devices that may cooperate - that undertake one or more measurements of a fuel intended for or introduced into the fuel line of a vehicle. The sensor(s) may be comprised within an integral unit suitably placed within or in close proximity to a fuel line or fuel storage tank within a vehicle. The sensor(s) may operate continuously (e.g. in real time) or may perform one or more measurements on a periodic basis. The sensor(s) will perform measurements on the fuel and may either output the measurements to an externally located processor, or may process the measurements within the sensor device. Suitably, the sensor may comprise one or more sensing means that contact the fuel directly in order to perform at least one measurement on the fuel.

As used herein, the term "engine control unit" (ECU) refers to device or system disposed within a vehicle that undertakes processing, monitoring and management of engine functions during operation of the vehicle. Typically the ECU will comprise a controller that may be in the form of one or more processors that that undertake said processing, monitoring and management of engine functions in response to a computer program. The controller may include, but not be limited to, a processor(s), computer(s), memory, storage, register(s), timing, interrupt(s), communication interfaces, and input/output signal interfaces, as well as combinations of the aforementioned. Typically the ECU is configured to be in communication with at least one sensor as defined above. Such communication may suitably occur via a direct link (e.g. through a wire, cable or optic fibre) or wirelessly (e.g. via radiofrequency such as Wi-Fi).

Unless otherwise defined, all technical and scientific terms used herein have the same meaning as commonly understood by one of ordinary skill in the art to which this invention belongs. For brevity the detailed operation of spark ignition and compression ignition engines is omitted as such operation is conventional.
In accordance with an embodiment of the invention, commercially available fuel survey data can be used to determine correlations between parameters that are directly measured by a fuel sensor and other parameters that cannot be subject to direct measurement. Fuel composition surveys are performed by national agencies (such as the US Environmental Protection Agency) as well as private organisations in all major countries in the world and provide detailed analytical results of fuel properties by geographical area (including by state, city or province). Annual fuel survey data may be obtained that covers all major geographical locations including Europe, the US, Canada, Brazil, Russia, India, China, and South Africa. In one embodiment of the present invention, the inventors have identified a series of correlations between directly measurable parameters and non-measurable parameters. The level of correlation is suitably greater than 50% probability, greater than 60% probability, greater than 70% probability, greater than 80% probability, and more suitably greater than 90% probability. According to a specific embodiment of the present invention a correlation is determined between density and viscosity in diesel fuel based upon fuel survey data in a given jurisdiction. This correlation can be determined by plotting a graph of density measurements obtained from the fuel survey against viscosity measurements so that a population of data points can be identified that represents the best statistical correlation for a given threshold of probability, for example in about 80% of all cases. In cases where the fuel sensor is able to measure only one parameter, such as density, the present invention therefore allows the density measurement taken to be 12 correlated with a high degree of probability to a viscosity value for fuels based upon the known data for that geographical region. The correlation step will typically occur in a processor located within the sensor or within the ECU.

Direct sensor measurements of density, aromatics content, and heating value/distillation properties can be correlated statistically with cetane number value. In addition derived parameter values can also be used to determine correlations with other fuel properties. By way of non-limiting example, the derived cetane number value for a given diesel fuel composition, may be correlated directly with measured parameters of aromatics content and heating value/distillation properties in order to determine cold flow parameters. In a similar fashion, measured parameters of density, and boiling point may be correlated with the derived parameter of viscosity in order to determine the lubricity of a given fuel composition. Table 1 sets out a number of directly measured parameters that can be correlated to parameters using fuel survey data. It will be appreciated that other parameters may also be derivable.

**Table 1**

| **Directly measured parameter using sensor data** | **Parameter derived by correlation with fuel survey data** |
|---|---|
| Density (kg/m³) | Viscosity (mm²/s) |
| Cetane Number | Aromatics (%m/m) |
| Biodiesel content (%m/m) | Cold flow |

Table 2 sets out a number of further parameters that may be derived by correlating directly measured parameters together with derived parameters (such as those shown in Table 1). It will be appreciated that other parameters will also be derivable.

**Table 2**

| **1** - **Directly measured parameter** | **2-Parameter derived by correlation with fuel survey data** | **Parameter derived by correlation with columns 1 and 2** |
|---|---|---|
| Density (kg/m³) | Viscosity (mm²/s) | Lubricity |
| Density (kg/m³), Cetane number | Aromatics (%m/m) | Distillation properties |
| Biodiesel content (%m/m) | Viscosity (mm²/s) | Lubricity |

Hence, it is apparent that by combining direct measurements obtained in real time from sensor data within the vehicle together with known fuel data information correlations can be made that allow for determinations of fuel quality across a number of parameters with a high degree of statistical significance.

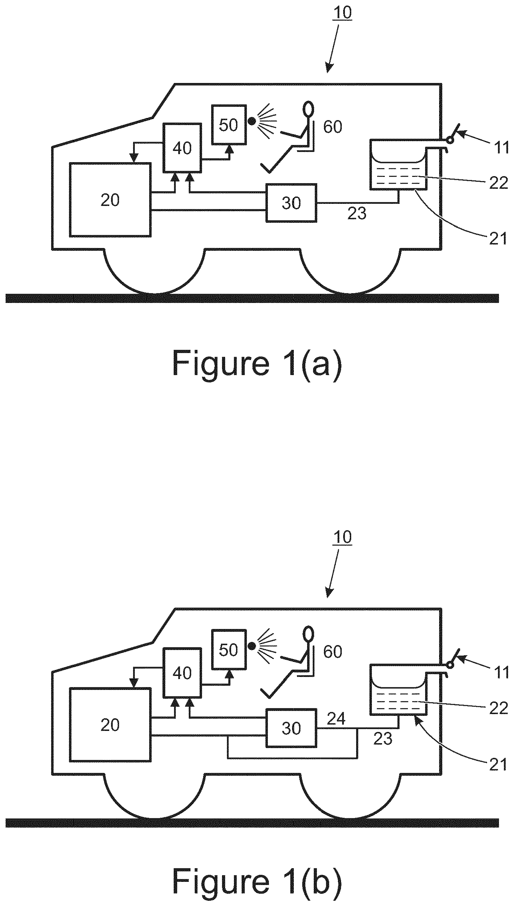

Referring to Figure 1, the present invention provides a method and system for alerting a vehicle operator 60 to a change in fuel quality during or after a refuelling event. Suitably the method alerts the operator 60 to a change in fuel quality such that it falls below a predetermined threshold and, thus, may be adverse to the continuing operation of the vehicle 10. During a refuelling event the method is initiated by the opening or removal of the fuel cap 11 (or opening of the fuel flap). New fuel is introduced into the fuel storage tank 21 where it may mix fully or partially with residual fuel so as to form a novel fuel composition 22. The storage tank 21 is in fluid communication with the engine 20 via the fuel line 23. In the event that the engine 20 is of the compression ignition type the fuel line 23 will typically lead from the tank 21 to a common rail compressor (not shown). The fuel composition 22 is taken into the fuel line 23 toward the engine 20. In one embodiment of the invention all of the fuel line 23 passes through a sensor 30 (see Figure 1 (a)). In an alternative embodiment of the invention the fuel line 23 is bifurcated and a side flow 24 of the fuel 22 is diverted into the sensor 30, whilst the main flow proceeds down the fuel line 23 (see Figure 1(b)). Fuel 22 passes through the sensor 30 which performs one or more measurements. Fuel 22 exits the sensor 30 - if diverted, the fuel 22 re-joins the main fuel line 23 - where it proceeds to the engine 20.

The sensor 30 is in communication with the ECU 40 and may undertake simple taking of measurements of the fuel 22 which are then transmitted to the ECU 40 for further processing. Alternatively, the sensor 30 may comprise full or partial processing functionality and, thus, output fuel quality information directly to the ECU 40. The form of the fuel quality information may vary but can range from detailed measured parameter information to calculated parameter information derived from the measured parameters.

Calculated fuel parameter information may be generated via a processor located within either the sensor 30 or the ECU 40, or by way of comparison to stored data and/or tables located within memory located within either the sensor 30 or the ECU 40. Where calculations of measurements are required the processor may operate according to one or more algorithms. The stored data may comprise information derived from or comprising fuel composition survey information for a given geographical region, typically the geographical region where the vehicle is located.

Fuel quality information is determined for the novel fuel composition 22 and may then be assessed to determine whether the fuel 22 meets minimum threshold levels established for the measurements. This assessment may occur within the ECU 40 or within a separate CPU comprised within the vehicle 10 (not shown). The assessment may be made by reference to stored data tables located within memory located within the ECU 40 or separate CPU. Alternatively, the assessment may be made via telemetry to a remotely located server (or cloud-based server) when the vehicle 10 is equipped with conventional transmitter/receiver apparatus able to operate on a wireless (Wi-Fi) or other mobile telecommunications network. Whether the fuel quality assessment is performed locally or remotely the outcome of the assessment results in a decision as to whether the fuel 22 complies with the threshold criteria necessary to ensure efficient and continued safe running of the vehicle 10. By way of example, the threshold may correspond with an established international standard for fuel quality such as European Standards EN590 (diesel fuel) and EN228 (gasoline fuel). If the decision is affirmative then the refuelling event may be logged within the ECU 40, separate CPU or at the remote server. If, however, the decision is negative then where a separate CPU or remote server are used these will typically communicate directly with the ECU 40 so that the decision is logged in memory and stored for future engine operation diagnostics during servicing or in the event of an engine failure. The ECU 40 will also issue an alert or notification regarding the quality of fuel 22 to the operator 60 via a human machine interface (HMI) 50. The alert may be in the form of a warning light, a warning message or icon displayed on a high level display front (HLDF), other LCD/LED screen or via a recorded audio message; or a more detailed diagnostic report concerning which measurements of fuel 22 have failed to meet the minimum criteria necessary for an affirmative determination of fuel quality. In extreme cases where continued operation of the vehicle 10 would likely cause irreparable and instantaneous damage to the engine it is an option for the ECU 40 to immobilise the engine 20 and prevent turnover. However, this is generally unlikely to occur under normal refuelling circumstances and instead the HMI 50 will more suitably provide information to the operator that the fuel 22 is of a quality that falls below the minimum threshold (i.e. is of low quality) so that the operator may make an informed choice at the next refuelling event whether to opt for fuel from a different supplier or source.

The vehicle 10 may be equipped optionally with global positioning system (GPS) technology (not shown). In instances where a negative determination of fuel quality occurs following a refuelling event, the geographical location of the refuelling event may be logged within the memory of the ECU 40 (or separate CPU or remote/cloud-based server, as appropriate). This can further enable the operator 60 to be reminded via the HMI 50 when next refuelling whether the chosen supplier is a known source of sub-standard quality fuel.

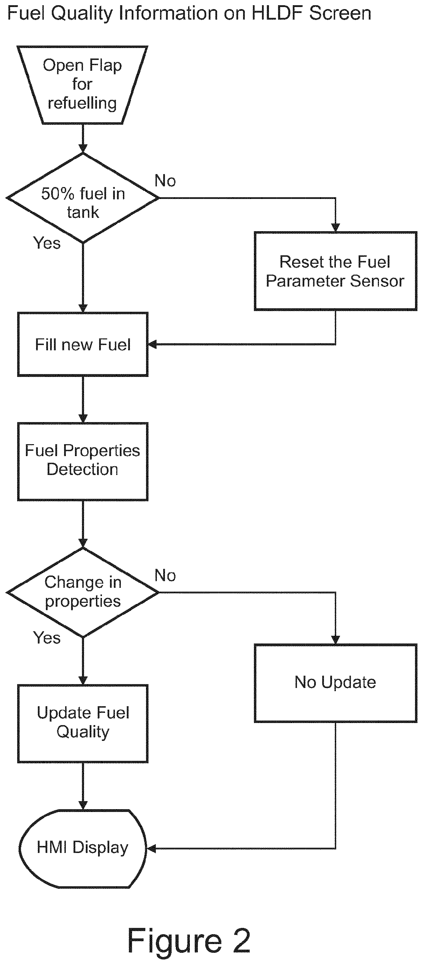

In figure 2 a flow diagram is provided that sets out a method of one embodiment of the present invention. The method may be initiated by opening of the fuel flap, removal of the fuel cap or by commencing a refuelling event. In this embodiment of the invention, a threshold is set for determination of a refuelling event that is effective to change the composition of the fuel held within the storage tank 21. The threshold shown in Figure 2 has been set at 50% (either by mass or volume), whereupon if there is greater than 50% of residual fuel remaining in the tank 21 the resultant combined fuel mix 22 is not considered to be a novel mixture and the system is reset. However, if there is less than 50% residual fuel in the tank 21 then the resultant combined fuel mix 22 is considered to be a novel mixture that should be subject to measurement. It will be understood that the residual fuel threshold need not be set only at 50% and may vary in a range of between about 20% and about 80%, suitably between about 30% and 70%, more suitably between about 40% and 60% (by mass or volume of fuel). The threshold may vary between countries/territories, as well as between winter and summer, and may be changed by accessing the ECU 40 (or sensor 30 or separate CPU where appropriate). The determination of residual fuel load in the tank 21 is made using conventional apparatus.

According to the method of the invention as set out in Figure 2, when the resultant combined fuel mix 22 is considered to be a novel mixture that should be subject to measurement the fuel 22 is subjected to a measurement step that detects the physical and chemical properties of the fuel 22. Suitably this step occurs within the sensor 30. If there is no substantial change in the properties the system is not updated, and no message is sent to the HMI display 50. If there is a substantial change in the properties the system is updated accordingly with information concerning the fuel quality and a message is sent to the HMI display 50.

The sensor 30 is suitably located within a low pressure region of the fuel supply system, typically upstream of the engine. As described previously, the sensor 30 provides measurement functionality for at least one physical or chemical parameter or property associated with the fuel 22.

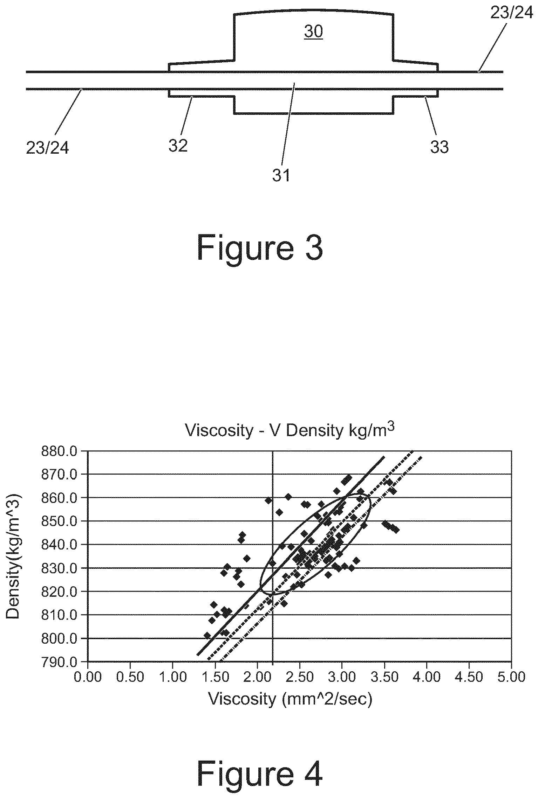

The sensor 30 may suitably comprise internal walls that define a chamber 31, the chamber 31 also comprising an inlet 32 and an outlet 33 (see Figure 3). As mentioned previously, the sensor 30 is located within a low pressure region of the fuel supply system, typically either directly on the fuel line 23, or side flow line 24 that is in fluid communication with the fuel line 23. The sensor 30 is positioned on the fuel line 23 (or side flow line 24) such that it intersects the line and allows for the fuel mix 22 to pass through the chamber via the inlet 32 and out towards the engine 20 via the outlet 33. Whilst Figure 3 shows for convenience that the flow path through the sensor 30 is substantially linear it will be appreciated that alternative configurations are also possible and the that path may be bifurcated or tortuous as required in order to obtain an arrangement that permits accurate and reliable measurement of fuel quality. The chamber 31, therefore, broadly defines an enclosed zone in which measurements of fuel quality may occur on the fuel mix 22.

In an embodiment of the invention the sensor 30 comprises an infrared spectrometer that allows for measurement of optical absorption of the fuel composition in the near infrared spectral range (i.e. 0.8-2.5 µm wavelength) as it passes through the chamber 31. Several chemical species and fuel characteristics that are determinative of fuel quality are known to have distinct fingerprints that can be detected in the near infrared spectra. Hence, the sensor 30 will comprise a measurement cell made of a suitable IR transparent material, such as glass, within the chamber 31. The measurements may be taken over several wavelengths in the near infrared so that different parameters may be obtained. Spectral data including absorption data may be interpreted directly within the sensor 30 or by the ECU 40 in order to determine the values of certain parameters. In addition, spectral data may be combined or subjected to further processing via one or more algorithms in order to determine other parameters. Again, this further processing may occur within a processor located in the sensor 30 or within the ECU 40. Hence, this embodiment of the invention allows for online sensor readings to be taken in real time for a specified period of time after a refuelling event.

In a specific embodiment of the invention the sensor 30 comprises a micro-opto-electromechanical system (MOEMS) that contains a microspectrometer that includes an integrated voltage controlled tunable interferometer together with a thermopile detector. This arrangement provides the advantage of allowing a large range of tunable wavelengths of only a few nm in the spectral band of 1 to 2 µm, thus, facilitating the taking of many measurements from the fuel mix 22 as it passes through the sensor 30.

Measurements made by the sensor 30 will be optimised for the particular type of engine 20 installed in the vehicle 10. Hence, for a spark ignition engine the sensor 30 will be optimised to perform measurements that are suited to determining the fuel quality of gasoline type fuels, including but not limited to one or more of the group consisting of:
- RON index
- Oxygen content (%wt)
- Volatility (e.g. Reid vapour pressure)
- Ethanol content (%wt)
- Density (kg/m³)

Whereas, for a compression ignition engine the sensor 30 will be optimised to perform measurements that are suited to determining the fuel quality of diesel type fuels, including but not limited to one or more of the group consisting of:
- Heating value (MJ/kg)
- Biodiesel content (%wt)
- Cetane number/index
- Density (kg/m³)
- Aromatics content (%wt)
- Sulphur content (%wt)
- Water content (%wt)
- FAME content (%wt)
- Wax content (%wt)

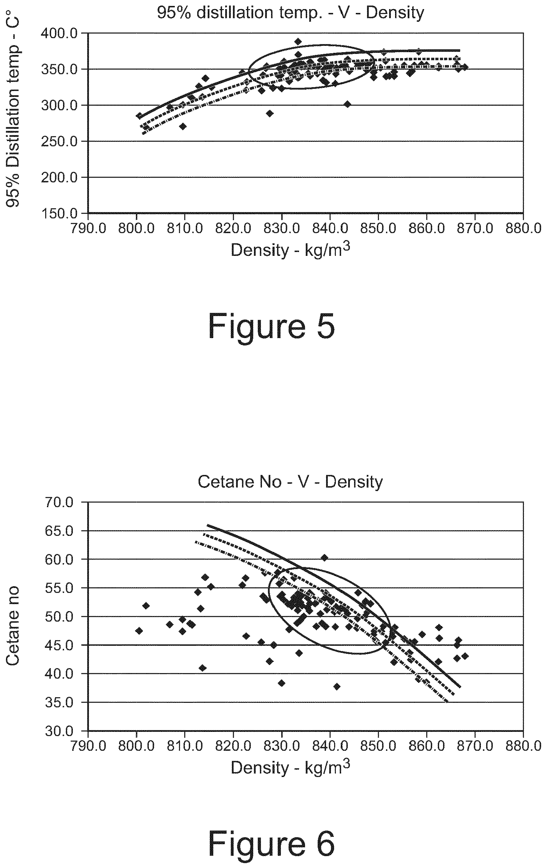

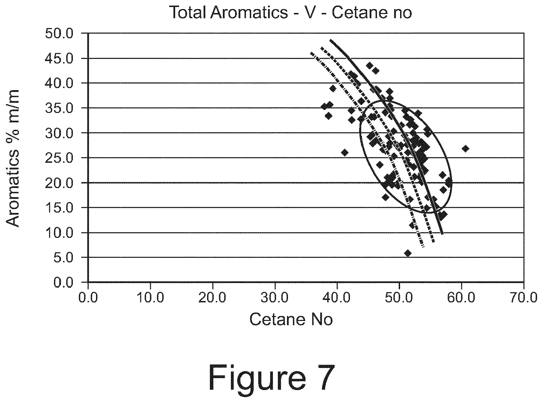

The sensor 30 may be configured to include a temperature sensor, for example by integrating an IR thermometer, a thermocouple, a thermopile or a thermistor into the sensor 30. Measurements of fuel 22 temperature along with the other stated parameters can be used to calculate other parameters including viscosity and lubricity of the fuel mix 22, such as via a prediction matrix or by reference to log table data stored within a memory in the sensor 30 or in the ECU 40. Such prediction matrices or log tables may be based upon reference data generated for near infrared spectra of fuel samples that comply with the relevant EU or US standards as well as market data obtained from fuel composition surveys. Hence, a reference database of fuel quality parameters can be built for each relevant parameter. This allows for a model to be built that enables prediction of fuel quality parameters from a so-called unknown sample - i.e. from the novel fuel mix 22.
In an example of the invention, density data are correlated against viscosity measurements from a commercially available diesel fuel composition survey. The correlation is in the form of a graph of density against viscosity for each fuel composition identified in the fuel survey for a given geographical area. Figure 4 shows such as graph with the region of highest correlation identified by the black oval. The nominal line of best fit is provided with positive and negative limits shown. Figures 5-7 show similar analyses for other correlated parameters in diesel fuel compositions. It is evident that reference tables can be compiled from these graphs based either on the lines of best fit or the identified regions of highest correlation in order to enable derivation of parameters from direct measurements of fuel properties. Alternatively, it is within the remit of the skilled person to produce one or more algorithms to enable calculation of the derived parameters based upon the known correlations shown in the accompanying graphs.
It is to be understood that embodiments of the invention are suitable for use with and within a wide variety of vehicle types and modes of operation.
Although particular embodiments of the invention have been disclosed herein in detail, this has been done by way of example and for the purposes of illustration only.

## Claims

1. A method for determining whether fuel quality falls below a predetermined quality threshold during or after a vehicle refuelling event, comprising the steps of:
a) determining that a novel fuel (22) has been introduced into a vehicle (10);
b) undertaking at least one measurement of the novel fuel (22) in order to determine at least one first parameter of the novel fuel (22), wherein the first parameter is associated with a physical and/or a chemical property of the fuel;
c) calculating the value of at least one second parameter based upon the first parameter determined in step b, the value of the at least one second parameter being determined by comparison to fuel survey information;
d) comparing the first and second parameters of the novel fuel (22) with corresponding threshold reference parameters in order to determine whether fuel quality falls below a predetermined quality threshold; and
e) providing an alert to the operator (60) of the vehicle (10) to indicate whether or if the fuel quality of the novel fuel (22) falls below the predetermined quality threshold,
wherein the at least one first parameter includes density and the at least one second parameter includes viscosity or aromatics, and/or
wherein the at least one first parameter includes cetane number and the at least one second parameter includes aromatics, and/or
wherein the at least one first parameter includes biodiesel content and the at least one second parameter includes viscosity.

2. The method of claim 1, wherein the method is initiated by opening of a fuel flap, removal of the fuel cap (11) or by commencing a refuelling event.

3. The method of claim 1 or claim 2, wherein the fuel comprises a gasoline.

4. The method of claim 1 or claim 2, wherein the fuel comprises a diesel fuel.

5. The method of claims 1 to 4, wherein the fuel comprises a biofuel component.

6. The method of any previous claim, wherein at least two first parameters are determined in step (b).

7. The method of claim 6, wherein a determination of whether the novel fuel (22) falls below the predetermined quality threshold comprises comparing each of said at least two first parameters with a respective plurality of threshold reference parameters.

8. The method of claim 7, wherein said plurality of threshold reference parameters are indicative of fuel which is in compliance with a regulatory standard.

9. The method as claimed in any preceding claim wherein the determination that a novel fuel (22) has been introduced into the vehicle (10) is made in response to a positive determination that the amount of fuel stored in the vehicle (10) has increased since a previous deactivation of the vehicle engine (20).

10. The method of any previous claim, wherein the comparison step (d) occurs within a microprocessor device (30, 40).

11. A system for use in a vehicle (10) which system determines whether fuel quality falls below a predetermined quality threshold during or after a vehicle refuelling event, the system (10) comprising:
(1) at least one sensor (30), the sensor (30) being adapted so as to undertake at least one measurement of a novel fuel (22) that has been introduced into the fuel system of the vehicle (10) in order to determine at least one first parameter of the novel fuel (22), wherein the first parameter is associated with a physical and/or a chemical property of the fuel;
(2) at least one processor (30, 40) that is in communication with the sensor (30), and which calculates the value of at least one second parameter based upon the first parameter, the value of the at least one second parameter being determined by comparison to fuel survey information, and compares the at least one first parameter and the at least one second parameter of the novel fuel (22) with corresponding threshold reference parameters in order to determine whether fuel quality falls below a predetermined quality threshold; and
(3) a display (50) that is in communication with the at least one processor (30, 40) for providing an alert to an operator (60) of the vehicle (10) to indicate whether or if the fuel quality of the novel fuel (22) falls below the predetermined quality threshold,
wherein the at least one first parameter includes density and the at least one second parameter includes viscosity or aromatics, and/or
wherein the at least one first parameter includes cetane number and the at least one second parameter includes aromatics, and/or
wherein the at least one first parameter includes biodiesel content and the at least one second parameter includes viscosity.

12. An engine control unit (40) comprising a control module that operates according to the method as set out in any of claims 1 to 10.

13. A vehicle (10) comprising a system according to claim 11 or an engine control unit (40) according to claim 12.

## Patentansprüche

1. Verfahren zum Bestimmen, ob Kraftstoffqualität während oder nach einem Fahrzeugbetankungsereignis unter eine vorgegebene Qualitätsschwelle fällt, die folgenden Schritte umfassend:
a) Bestimmen, dass ein neuer Kraftstoff (22) in ein Fahrzeug (10) eingeleitet wurde;
b) Durchführen wenigstens einer Messung des neuen Kraftstoffs (22), um wenigstens einen ersten Parameter des neuen Kraftstoffs (22) zu bestimmen, wobei der erste Parameter mit einer physikalischen und/oder einer chemischen Eigenschaft des Kraftstoffs verknüpft ist;
c) Berechnen des Werts wenigstens eines zweiten Parameters auf Grundlage des in Schritt b bestimmten ersten Parameters, wobei der Wert des wenigstens einen zweiten Parameters durch Vergleichen mit Kraftstoffmessinformationen bestimmt wird;
d) Vergleichen des ersten und des zweiten Parameters des neuen Kraftstoffs (22) mit entsprechenden Schwellenreferenzparametern, um zu bestimmen, ob Kraftstoffqualität unter eine vorgegebene Qualitätsschwelle fällt; und
e) Bereitstellen einer Warnung an den Betreiber (60) des Fahrzeugs (10), um anzugeben, ob oder falls die Kraftstoffqualität des neuen Kraftstoffs (22) unter die vorgegebene Qualitätsschwelle fällt,
wobei der wenigstens eine erste Parameter Dichte einschließt und der wenigstens eine zweite Parameter Viskosität oder Aromate einschließt, und/oder
wobei der wenigstens eine erste Parameter Cetanzahl einschließt und der wenigstens eine zweite Parameter Aromate einschließt, und/oder
wobei der wenigstens eine erste Parameter Biodieselgehalt einschließt und der wenigstens eine zweite Parameter Viskosität einschließt.

2. Verfahren nach Anspruch 1, wobei das Verfahren durch Öffnen einer Tankklappe, Entfernen des Tankverschlusses (11) oder durch Beginnen eines Betankungsereignisses eingeleitet wird.

3. Verfahren nach Anspruch 1 oder 2, wobei der Kraftstoff ein Benzin umfasst.

4. Verfahren nach Anspruch 1 oder 2, wobei der Kraftstoff einen Dieselkraftstoff umfasst.

5. Verfahren nach Anspruch 1 oder 4, wobei der Kraftstoff eine Biokraftstoffkomponente umfasst.

6. Verfahren nach einem der vorhergehenden Ansprüche, wobei wenigstens zwei erste Parameter in Schritt (b) bestimmt werden.

7. Verfahren nach Anspruch 6, wobei ein Bestimmen, ob der neue Kraftstoff (22) unter die vorgegebene Qualitätsschwelle fällt, ein Vergleichen jedes der wenigstens zwei ersten Parameter mit einer entsprechenden Anzahl von Schwellenreferenzparametern umfasst.

8. Verfahren nach Anspruch 7, wobei die mehreren Schwellenreferenzparameter einen Kraftstoff angeben, der einen Regulierungsstandard erfüllt.

9. Verfahren nach einem der vorhergehenden Ansprüche, wobei das Bestimmen, dass ein neuer Kraftstoff (22) in das Fahrzeug (10) eingeleitet wurde, in Reaktion auf ein positives Bestimmen erfolgt, dass die Menge an in dem Fahrzeug (10) gespeichertem Kraftstoff seit einer vorausgehenden Deaktivierung des Fahrzeugmotors (20) gestiegen ist.

10. Verfahren nach einem der vorhergehenden Ansprüche, wobei der Vergleichsschritt (d) in einer Mikroprozessorvorrichtung (30, 40) stattfindet.

11. System für die Verwendung in einem Fahrzeug (10), wobei das System bestimmt, ob Kraftstoffqualität während oder nach einem Fahrzeugbetankungsereignis unter eine vorgegebene Qualitätsschwelle fällt, wobei das System (10) Folgendes umfasst:
(1) wenigstens einen Sensor (30), wobei der Sensor (30) angepasst ist, um wenigstens eine Messung eines neuen Kraftstoffs (22), der in das Kraftstoffsystem des Fahrzeugs (10) eingeleitet wurde, durchzuführen, um wenigstens einen ersten Parameter des neuen Kraftstoffs (22) zu bestimmen, wobei der erste Parameter mit einer physikalischen und/oder einer chemischen Eigenschaft des Kraftstoffs verknüpft ist;
(2) wenigstens einen Prozessor (30, 40), der in Kommunikation mit dem Sensor (30) ist, und der den Wert wenigstens eines zweiten Parameters auf Grundlage des ersten Parameters berechnet, wobei der Wert des wenigstens einen zweiten Parameters durch Vergleichen mit Kraftstoffmessinformationen bestimmt wird, und den wenigstens einen ersten Parameter und den wenigstens einen zweiten Parameter des neuen Kraftstoffs (22) mit entsprechenden Schwellenreferenzparametern vergleicht, um zu bestimmen, ob Kraftstoffqualität unter eine vorgegebene Qualitätsschwelle fällt; und
(3) eine Anzeige (50), die in Kommunikation mit dem wenigstens einen Prozessor (30, 40) ist, um eine Warnung an einen Betreiber (60) des Fahrzeugs (10) bereitzustellen, um anzugeben, ob oder falls die Kraftstoffqualität des neuen Kraftstoffs (22) unter die vorgegebene Qualitätsschwelle fällt,
wobei der wenigstens eine erste Parameter Dichte einschließt und der wenigstens eine zweite Parameter Viskosität oder Aromate einschließt, und/oder
wobei der wenigstens eine erste Parameter Cetanzahl einschließt und der wenigstens eine zweite Parameter Aromate einschließt, und/oder
wobei der wenigstens eine erste Parameter Biodieselgehalt einschließt und der wenigstens eine zweite Parameter Viskosität einschließt.

12. Motorsteuergerät (40), das ein Steuermodul umfasst, das gemäß dem Verfahren nach einem der Ansprüche 1 bis 10 betrieben wird.

13. Fahrzeug (10), das ein System nach Anspruch 11 oder ein Motorsteuergerät (40) nach Anspruch 12 umfasst.

## Revendications

1. Procédé permettant de déterminer si la qualité du carburant est inférieure à un seuil de qualité prédéterminé pendant ou après une opération de ravitaillement d'un véhicule en carburant, comprenant les étapes consistant à :
a) constater qu'un nouveau carburant (22) a été introduit dans un véhicule (10) ;
b) réaliser au moins une mesure du nouveau carburant (22) afin de déterminer au moins un premier paramètre du nouveau carburant (22), le premier paramètre étant associé à une propriété physique et/ou chimique du carburant ;
c) calculer la valeur d'au moins un second paramètre sur la base du premier paramètre déterminé à l'étape (b), la valeur de l'au moins un second paramètre étant déterminée par comparaison à des données d'analyse de carburant ;
d) comparer les premier et second paramètres du nouveau carburant (22) avec des paramètres de référence de seuil correspondants afin de déterminer si la qualité du carburant est inférieure à un seuil de qualité prédéterminé ; et
e) adresser une notification au conducteur (60) du véhicule (10) pour lui indiquer si la qualité de carburant du nouveau carburant (22) est inférieure au seuil de qualité prédéterminé,
dans lequel l'au moins un premier paramètre comprend la densité et l'au moins un second paramètre comprend la viscosité ou la teneur en composés aromatiques, et/ou
dans lequel l'au moins un premier paramètre comprend l'indice de cétane et l'au moins un second paramètre comprend la teneur en composés aromatiques, et/ou
dans lequel l'au moins un premier paramètre comprend la teneur en biodiesel et l'au moins un second paramètre comprend la viscosité.

2. Procédé selon la revendication 1, dans lequel le procédé est initié par l'ouverture de la trappe à carburant, le retrait du bouchon du réservoir à carburant (11) ou par le début de l'opération de ravitaillement en carburant.

3. Procédé selon la revendication 1 ou la revendication 2, dans lequel le carburant comprend une essence.

4. Procédé selon la revendication 1 ou la revendication 2, dans lequel le carburant comprend un carburant diesel.

5. Procédé selon les revendications 1 à 4, dans lequel le carburant comprend un constituant de biocarburant.

6. Procédé selon l'une quelconque des revendications précédentes, dans lequel au moins deux premiers paramètres sont déterminés à l'étape (b).

7. Procédé selon la revendication 6, dans lequel la détermination du fait que la qualité du nouveau carburant (22) est ou non inférieure au seuil de qualité prédéterminé comprend la comparaison de chacun desdits au moins deux premiers paramètres avec une pluralité respective de paramètres de référence de seuil.

8. Procédé selon la revendication 7, dans lequel ladite pluralité de paramètres de référence de seuil indiquent si un carburant est ou non conforme à une norme réglementaire.

9. Procédé selon l'une quelconque des revendications précédentes, dans lequel la constatation du fait qu'un nouveau carburant (22) a été introduit dans le véhicule (10) intervient en réponse à une détermination positive que la quantité de carburant stockée dans le véhicule (10) a augmenté depuis une désactivation antérieure du moteur du véhicule (20).

10. Procédé selon l'une quelconque des revendications précédentes, dans lequel l'étape de comparaison (d) s'effectue à l'intérieur d'un dispositif à microprocesseur (30, 40).

11. Système conçu pour être utilisé dans un véhicule (10), lequel système détermine si la qualité du carburant est inférieure à un seuil de qualité prédéterminé pendant ou après une opération de ravitaillement du véhicule en carburant, le système (10) comprenant :
(1) au moins un capteur (30), le capteur (30) étant adapté pour réaliser au moins une mesure sur un nouveau carburant (22) introduit dans le système de carburant du véhicule (10) afin de déterminer au moins un premier paramètre du nouveau carburant (22), le premier paramètre étant associé à une propriété physique et/ou chimique du carburant ;
(2) au moins un processeur (30, 40) communiquant avec le capteur (30), et qui calcule la valeur d'au moins un second paramètre sur la base du premier paramètre, la valeur de l'au moins un second paramètre étant déterminée par comparaison à des données d'analyse de carburant, et compare l'au moins un premier paramètre et l'au moins un second paramètre du nouveau carburant (22) avec des paramètres de référence de seuil correspondants afin de déterminer si la qualité du carburant est inférieure à un seuil de qualité prédéterminé ; et
(3) un écran (50) communiquant avec l'au moins un processeur (30, 40) pour adresser une notification au conducteur (60) du véhicule (10) et lui indiquer si la qualité de carburant du nouveau carburant (22) est inférieure au seuil de qualité prédéterminé,
dans lequel l'au moins un premier paramètre comprend la densité et l'au moins un second paramètre comprend la viscosité ou la teneur en composés aromatiques, et/ou
dans lequel l'au moins un premier paramètre comprend l'indice de cétane et l'au moins un second paramètre comprend la teneur en composés aromatiques, et/ou
dans lequel l'au moins un premier paramètre comprend la teneur en biodiesel et l'au moins un second paramètre comprend la viscosité.

12. Unité de commande de moteur (40) comprenant un module de commande qui fonctionne conformément au procédé selon l'une quelconque des revendications 1 à 10.

13. Véhicule (10) comprenant un système selon la revendication 11 ou une unité de commande de moteur (40) selon la revendication 12.
